# EUROPÄISCHE PATENTANMELDUNG

(11) **EP 1 086 687 A2**
(43) Veröffentlichungstag der Anmeldung: **28.03.2001**
(21) Anmeldenummer: 00119870.4
(22) Anmeldetag: 13.09.2000
(51) Int. Cl.: A61K 7/48

(54) **Zubereitungen vom Emulsionstyp Wasser-in-Öl mit einem Gehalt an einem oder mehreren Siliconölen sowie einem oder mehreren kationischen Polymeren**

(30) Priorität: 22.09.1999 DE 19945283
(71) Anmelder: Beiersdorf AG, 20245 Hamburg (DE)
(72) Erfinder: Bleckmann, Andreas, 22926 Ahrensburg (DE); Kröpke, Rainer, 22869 Schenefeld (DE); Kovacevic, Melanie, 20251 Hamburg (DE); Schneider, Günther, Dr., 22607 Hamburg (DE); Riedel, Heidi, 22529 Hamburg (DE)

(57) **Zusammenfassung**

Wasser-in-Öl-Emulsionen
(a) eines Gehaltes an Wasser und gegebenenfalls wasserlöslichen Substanzen
(b) und eines Gehaltes an Lipiden und gegebenenfalls lipophilen Bestandteilen von insgesamt mindestens 20 Gew.-%, jeweils bezogen auf das Gesamtgewicht der Zubereitungen,
   enthaltend:
(c) wenigstens ein Siliconöl in einem Gehalt von mindestens 7,5 Gew.-%, bevorzugt mindestens 10 Gew.-%, bezogen auf das Gesamtgewicht der Zubereitungen
(e) mindestens eine grenzflächenaktive Substanz, gewählt aus der Gruppe der Alkylmethiconcopolyole und/oder Alkyl-Dimethiconcopolyole,
(e) ein oder mehrere kationische Polymere.

## Beschreibung

Die vorliegende Erfindung betrifft kosmetische und dermatologische Emulsionen vom Emulsionstyp Wasser-in-Öl mit einem Gehalt an einem oder mehreren Siliconölen sowie einem oder mehreren kationischen Polymeren, insbesondere hautpflegende kosmetische und dermatologische Emulsionen. In einer vorteilhaften Ausführungsform betrifft die vorliegende Erfindung Anwendungen, welche es erlauben, kosmetisch elegante Zubereitungen, insbesondere Emulsionen, mit hervorragenden sensorischen und hautpflegenden Eigenschaften herzustellen.

Die Haut ist das größte Organ des Menschen. Unter ihren vielen Funktionen (beispielsweise zur Wärmeregulation und als Sinnesorgan) ist die Barrierefunktion, die das Austrocknen der Haut (und damit letztlich des gesamten Organismus) verhindert, die wohl wichtigste. Gleichzeitig wirkt die Haut als Schutzeinrichtung gegen das Eindringen und die Aufnahme von außen kommender Stoffe. Bewirkt wird diese Barrierefunktion durch die Epidermis, welche als äußerste Schicht die eigentliche Schutzhülle gegenüber der Umwelt bildet. Mit etwa einem Zehntel der Gesamtdicke ist sie gleichzeitig die dünnste Schicht der Haut.

Die Epidermis ist ein stratifiziertes Gewebe, in dem die äußere Schicht, die Hornschicht (Stratum corneum), den für die Barrierefunktion bedeutenden Teil darstellt. Das heute in der Fachwelt anerkannte Hautmodell von Elias (*P. M. Elias, Structure and Function of the Stratum Corneum Permeability Barrier, Drug Dev. Res. **13**, 1988, 97-105*) beschreibt die Homschicht als Zwei-Komponenten-System, ähnlich einer Ziegelsteinmauer (Ziegelstein-Mörtel-Modell). In diesem Modell entsprechen die Hornzellen (Korneozyten) den Ziegelsteinen, die komplex zusammengesetzte Lipidmembran in den Interzellularräumen entspricht dem Mörtel. Dieses System stellt im wesentlichen eine physikalische Barriere gegen hydrophile Substanzen dar, kann aber aufgrund seiner engen und mehrschichtigen Struktur gleichermaßen auch von lipophilen Substanzen nur schwer passiert werden.

Die vorliegende Erfindung betrifft in einer besonderen Ausführungsform kosmetische oder pharmazeutische Zubereitungen mit vermindertem Klebrigkeitsgefühl, Verfahren zu ihrer Herstellung sowie die Verwendung von Wirkstoffen zur Herabminderung des Klebrigkeitsgefühles kosmetischer Zubereitungen.

Außer ihrer Barrierewirkung gegen externe chemische und physikalische Einflüsse tragen die epidermalen Lipide auch zum Zusammenhalt der Hornschicht bei und haben Einfluß auf die Hautglätte. Im Gegensatz zu den Talgdrüsenlipiden, die keinen geschlossenen Film auf der Haut ausbilden, sind die epidermalen Lipide über die gesamte Hornschicht verteilt.

Das äußerst komplexe Zusammenwirken der feuchtigkeitsbindenden Substanzen und der Lipide der oberen Hautschichten ist für die Regulation der Hautfeuchte sehr wichtig. Daher enthalten Kosmetika in der Regel, neben ausgewogenen Lipidabmischungen und Wasser, wasserbindende Substanzen.

Neben der chemischen Zusammensetzung ist jedoch auch das physikalische Verhalten dieser Substanzen von Bedeutung. Daher ist die Entwicklung von sehr gut bioverträglichen Emulgatoren bzw. Tensiden wünschenswert. Damit formulierte Produkte unterstützen die flüssigkristalline Organisation der Interzellularlipide des Stratum Corneums und verbessern so die Barriereeigenschaften der Hornschicht. Besonders vorteilhaft ist es, wenn deren Molekülbestandteile aus natürlicherweise in der Epidermis vorkommenden Substanzen bestehen.

Unter kosmetischer Hautpflege ist in erster Linie zu verstehen, daß die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) gestärkt oder wiederhergestellt wird.

Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliche Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Medizinische topische Zusammensetzungen enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Übliche kosmetische Darreichungsformen sind Emulsionen. Darunter versteht man im allgemeinen ein heterogenes System aus zwei miteinander nicht oder nur begrenzt mischbaren Flüssigkeiten, die üblicherweise als Phasen bezeichnet werden. Die eine liegt dabei in Form von Tröpfchen vor (dispere oder innere Phase), während die andere Flüssigkeit eine kontinuierliche (kohärente oder innere Phase) bildet. Seltenere Darreichungsformen sind multiple Emulsionen, also solche, welche in den Tröpfchen der dispergierten (oder diskontinuierlichen) Phase ihrerseits Tröpfchen einer weiteren dispergierten Phase enthalten, z.B. W/O/W-Emulsionen und O/W/O-Emulsionen.

Natürlich ist dem Fachmann eine Vielzahl von Möglichkeiten bekannt, stabile W/O-Zubereitungen zur kosmetischen oder dermatologischen Anwendung zu formulieren, beispielsweise in Form von Cremes und Salben, die im Bereich von Raum- bis Hauttemperatur streichfähig sind, oder als Lotionen und Milche, die in diesem Temperaturbereich eher fließfähig sind.

Die Formulierung stabiler sowie sensorisch attraktiver Wasser-in-Öl-Formulierungen mit höherem Silikongehalt (beispielsweise > 10 Gew.-%) gestaltet sich derzeit als nach wie vor schwierig, da diese Emulsionen einerseits keine ausreichenden Lagerstabilitäten (> 3 Monate) aufweisen und zum anderen die Verwendung hoher Mengen an Silikonölen (insbesondere an Di- und Cyclomethiconen) eine zwar angenehme, aber sehr reichhaltige Sensorik (fettiges, öliges Hautgefühl mit viel Rückstand beim Verteilen auf der Haut) zur Folge hat.

Überraschend hat sich gezeigt, daß Wasser-in-Öl-Emulsionen
(a) eines Gehaltes an Wasser und gegebenenfalls wasserlöslichen Substanzen
(b) und eines Gehaltes an Lipiden und gegebenenfalls lipophilen Bestandteilen von insgesamt mindestens 20 Gew.-%, jeweils bezogen auf das Gesamtgewicht der Zubereitungen,
   enthaltend:
(c) wenigstens ein Siliconöl in einem Gehalt von mindestens 7,5 Gew.-%, bevorzugt mindestens 10 Gew.-%, bezogen auf das Gesamtgewicht der Zubereitungen
(d) mindestens eine grenzflächenaktive Substanz, gewählt aus der Gruppe der Alkylmethiconcopolyole und/oder Alkyl-Dimethiconcopolyole,
(e) ein oder mehrere kationische Polymere
   den Nachteilen des Standes der Technik abhelfen.

Erstaunlicherweise sind die erfindungsgemäßen Zubereitungen in der Anwendung auf der Haut äußerst angenehm und zeichnen sich durch sehr hohe kosmetische Eleganz aus. Es ist mit den Mitteln, die dem Fachmanne ohne weiteres erfinderisches Zutun geläufig sind, nahezu beliebige Viskositäten erreichbar, so daß die vorliegende Erfindung beispielsweise als fließfähige Darreichungsform (etwa eine Lotion) oder als halbfeste bis feste Zubereitung (etwa als eine Crème) ausgestaltet werden kann.

Erfindungsgemäß können die Siliconemulgatoren vorteilhaft aus der Gruppe grenzflächenaktive Substanzen aus der Gruppe der Alkylmethiconcopolyole und/oder Alkyl-Dimethiconcopolyole gewählt werden, insbesondere aus der Gruppe der Verbindungen, welche gekennzeichnet sind durch die folgende chemische Struktur: 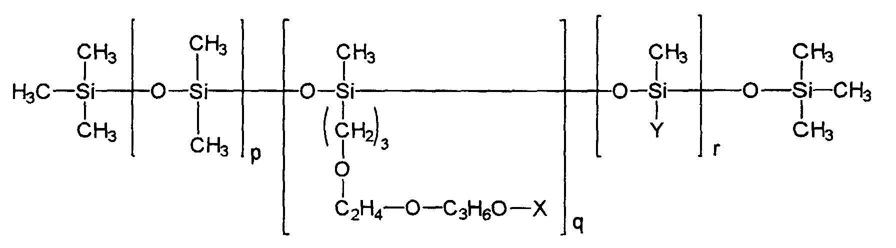 bei welcher X und Y unabhängig voneinander gewählt werden aus der Gruppe H sowie der verzweigten und unverzweigten Alkylgruppen, Acylgruppen und Alkoxygruppen mit 1 - 24 Kohlenstoffatomen, p eine Zahl von 0 - 200 darstellt, q eine Zahl von 1 - 40 darstellt, und r eine Zahl von 1 - 100 darstellt.

Ein Beispiel für besonders vorteilhaft im Sinne der vorliegenden Erfindung zu verwendende Silikonemulgatoren sind Dimethiconcopolyole, welche von der Gesellschaft Th.Goldschmidt AG unter den Warenbezeichnungen ABIL® B 8842, ABIL® B 8843, ABIL® B 8847, ABIL® B 8851, ABIL® B 8852, ABIL® B 8863, ABIL® B 8873 und ABIL® B 88183 verkauft werden.

Ein weiteres Beispiel für besonders vorteilhaft im Sinne der vorliegenden Erfindung zu verwendende grenzflächenaktiven Substanzen ist das Cetyl Dimethiconcopolyol, welches von der Gesellschaft Th.Goldschmidt AG unter der Warenbezeichnung ABIL® EM 90 verkauft wird.

Ein weiteres Beispiel für besonders vorteilhaft im Sinne der vorliegenden Erfindung zu verwendende grenzflächenaktiven Substanzen ist das Cyclomethicon Dimethiconcopolyol, welches von der Gesellschaft Th.Goldschmidt AG unter der Warenbezeichnung ABIL® EM 97 verkauft wird.

Weiterhin hat sich als ganz besonders vorteilhaft der Emulgator Laurylmethiconcopolyol herausgestellt, welcher unter der Warenbezeichnung Dow Corning® 5200 Formulation Aid von der Gesellschaft Dow Corning Ltd. erhältlich ist.

Die Gesamtmenge an erfindungsgemäß verwendeten Silikonemulgatoren in den erfindungsgemäßen kosmetischen oder dermatologischen Zubereitungen wird vorteilhaft aus dem Bereich von 0,1 - 10,0 Gew.-%, bevorzugt 0,5 - 5,0 Gew.-% gewählt, bezogen auf das Gesamtgewicht der Zubereitungen.

Überraschend hat sich insbesondere gezeigt, daß durch den Zusatz von 0,01 bis 10% (bevorzugt 0,25 - 1,25 %) geeigneter kationischer Polymere stabile, feste bis halbfeste und/oder fließfähige Wasser-in-Silicon-Emulsionen" hergestellt werden können, die über hervorragende sensorische Eigenschaften verfügen.

Geeignete kationische Polymere sind beispielsweise kationische Cellulosederivate (z.B. Polymer JR 400® von Amerchol), kationische Stärke, Copolymere von Diallylammoniumsalzen und Acrylamiden, quaternisierte Vinylpyrrolidon/Vinyl-imadazol-Polymere (z.B. Luviquat® von der BASE), Kondensationsprodukte von Polyglycolen und Aminen, quaternisierte Kollagenpolypeptide (z.B. Lamequat® L von Grünau-Henkel), quaternisierte Weizenpolypeptide, Polyethylenimin, kationische Silikonpolymere, Copolymere der Adipinsäure mit Dimethylaminohydroxypropyldietylentriamin, Copolymere der Acrylsäure mit Dimethyldiallylamoniumchlorid (z.B. Merquat®550 von Chemviron), Polyaminopolyamide, kationische Chitinderivate, kationischer Guar-Gum (z.B. Jaguar® CBS von Hoechst Celanese), quaternisierte Ammoniumsalz-Polymere (z.B. Mirapol® AD-1 von Miranol) sowie kationische Biopolymere wie z.B. Chitosan (mittleres Molekulargewicht von 50.000 bis 2.000.000 g/mol [bestimmt mittels Gelpermeationschromatographie] und einem Deacylierungsgrad von 10 bis 99% [bestimmt mittels ¹H-NMR]).

Es wird bevorzugt, die Ölphase der erfindungsgemäßen Zubereitungen zu mindestens 7,5 Gew.-%, bezogen auf das Gesamtgewicht der Zubereitungen, aus der Gruppe der cyclischen und/oder linearen Silicone zu wählen, welche im Rahmen der vorliegenden Offenbarung auch als 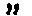 Siliconöle" bezeichnet werden. Solche Silicone oder Siliconöle können als Monomere vorliegen, welche in der Regel durch Strukturelemente charakterisiert sind, wie folgt:

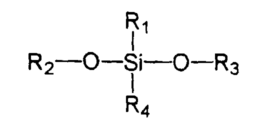

Als erfindungsgemäß vorteilhaft einzusetzenden linearen Silicone mit mehreren Siloxyleinheiten werden im allgemeinen durch Strukturelemente charakterisiert wie folgt: 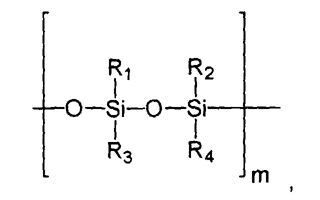 wobei die Siliciumatome mit gleichen oder unterschiedlichen Alkylresten und/oder Arylresten substituiert werden können, welche hier verallgemeinernd durch die Reste R₁ - R₄ dargestellt sind (will sagen, daß die Anzahl der unterschiedlichen Reste nicht notwendig auf bis zu 4 beschränkt ist). m kann dabei Werte von 2 - 200.000 annehmen.

Erfindungsgemäß vorteilhaft einzusetzende cyclische Silicone werden im allgemeinen durch Strukturelemente charakterisiert, wie folgt 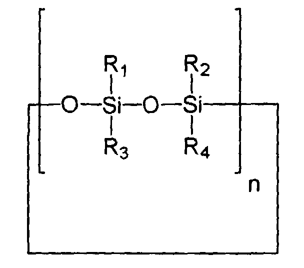 wobei die Siliciumatome mit gleichen oder unterschiedlichen Alkylresten und/oder Arylresten substituiert werden können, welche hier verallgemeinernd durch die Reste R₁ - R₄ dargestellt sind (will sagen, daß die Anzahl der unterschiedlichen Reste nicht notwendig auf bis zu 4 beschränkt ist). n kann dabei Werte von 3/2 bis 20 annehmen. Gebrochene Werte für n berücksichtigen, daß ungeradzahlige Anzahlen von Siloxylgruppen im Cyclus vorhanden sein können.

Vorteilhaft wird Phenyltrimethicon als Siliconöl gewählt. Auch andere Silikonöle, beispielsweise Dimethicon, Phenyldimethicon, Cyclomethicon (Octamethylcyclotetrasiloxan) beispielsweise Hexamethylcyclotrisiloxan, Polydimethylsiloxan, Poly(methylphenylsiloxan), Cetyldimethicon, Behenoxydimethicon sind vorteilhaft im Sinne der vorliegenden Erfindung zu verwenden.

Vorteilhaft sind ferner Mischungen aus Cyclomethicon und Isotridecylisononanoat, sowie solche aus Cyclomethicon und 2-Ethylhexylisostearat.

Es ist aber auch vorteilhaft, Silikonöle ähnlicher Konstitution wie der vorstehend bezeichneten Verbindungen zu wählen, deren organische Seitenketten derivatisiert, beispielsweise polyethoxyliert und/oder polypropoxyliert sind. Dazu zählen beispielsweise Polysiloxan-polyalkyl-polyether-copolymere wie das Cetyl-Dimethicon-Copolyol, das (Cetyl-Dimethicon-Copolyol (und) Polyglyceryl-4-Isostearat (und) Hexyllaurat)

Im Rahmen der vorliegenden Offenbarung wird als Oberbegriff für Fette, Öle, Wachse und dergleichen gelegentlich der Ausdruck 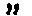 Lipide" verwendet, wie dem Fachmanne durchaus geläufig ist. Auch werden die Begriffe 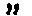 Ölphase" und 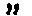 Lipidphase" synonym angewandt.

Vorteilhaft können weitere Bestandteile der Lipidphase gewählt werden aus der Gruppe der polaren und der unpolaren Lipidkomponenten, beispielsweise gewählt aus der Gruppe Vaseline (Petrolatum), Paraffinöl und Polyolefine. Unter den Polyolefinen sind Polydecene und Hydriertes Polyisobutene die bevorzugten Substanzen.

Die Ölphase kann im Sinne der vorliegenden Erfindung ferner vorteilhaft Substanzen enthalten, gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, wie z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlange von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Erfindungsgemäß vorteilhaft in der Ölphase einzusetzende Fett- und/oder Wachskomponenten können aus der Gruppe der pflanzlichen Wachse, tierischen Wachse, Mineralwachse und petrochemischen Wachse gewählt werden. Erfindungsgemäß günstig sind beispielsweise Candelillawachs, Carnaubawachs, Japanwachs, Espartograswachs, Korkwachs, Guarumawachs, Reiskeimölwachs, Zuckerrohrwachs, Beerenwachs, Ouricurywachs, Montanwachs, Jojobawachs, Shea Butter, Bienenwachs, Schellackwachs, Walrat, Lanolin (Wollwachs), Bürzelfett, Ceresin, Ozokerit (Erdwachs), Paraffinwachse und Mikrowachse.

Weitere vorteilhafte Fett- und/oder Wachskomponenten sind chemisch modifizierte Wachse und synthetische Wachse, wie beispielsweise die unter den Handelsbezeichnungen Syncrowax HRC (Glyceryltribehenat), Syncrowax HGLC (C₁₆₋₃₆ -Fettsäuretriglycerid) und Syncrowax AW 1C (C₁₈₋₃₆ -Fettsäure) bei der CRODA GmbH erhältlichen sowie Montanesterwachse, Sasolwachse, hydrierte Jojobawachse, synthetische oder modifizierte Bienenwachse (z. B. Dimethicon Copolyol Bienenwachs und/oder C₃₀₋₅₀ -Alkyl Bienenwachs), Polyalkylenwachse, Polyethylenglykolwachse, aber auch chemisch modifizierte Fette, wie z. B. hydrierte Pflanzenöle (beispielsweise hydriertes Ricinusöl und/oder hydrierte Cocosfettglyceride), Triglyceride, wie beispielsweise Trihydroxystearin, Fettsäuren, Fettsäureester und Glykolester, wie beispielsweise C₂₀₋₄₀-Alkylstearat, C₂₀₋₄₀-Alkylhydroxystearoylstearat und/oder Glykolmontanat. Weiter vorteilhaft sind auch bestimmte Organosiliciumverbindungen, die ähnliche physikalische Eigenschaften aufweisen wie die genannten Fett- und/oder Wachskomponenten, wie beispielsweise Stearoxytrimethylsilan.

Erfindungsgemäß können die Fett- und/oder Wachskomponenten sowohl einzeln als auch im Gemisch vorliegen.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als Lipidkomponente der Ölphase einzusetzen.

Von den Kohlenwasserstoffen sind Paraffinöl, hydrierte Polyolefine (z.B. hydriertes Polyisobuten) Squalan und Squalen vorteilhaft im Sinne der vorliegenden Erfindung zu verwenden.

Erfindungsgemäß vorteilhaft kann die Ölphase auch Bestandteile umfassen, gewählt aus der Gruppe der klassischen Fette, also im wesentlichen Triglyceriden.

Die erfindungsgemäßen Öle werden vorzugsweise gewählt aus der Gruppe der Triglyceride folgender Struktur: 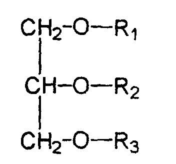 wobei R₁, R₂ und R₃ unabhängig voneinander gewählt werden aus der Gruppe der verzweigten und unverzweigten, Alkylcarboxyl- bzw. Alkenylcarboxylgruppen mit 12 bis 24 Kohlenstoffatomen. Es ist gegebenenfalls vorteilhaft, wenn eine oder mehrere aliphatische Wasserstoffatome der Alkylcarboxyl- bzw. Alkenylcarboxylgruppen durch Hydroxylgruppen substituiert sind.

Insbesondere ist vorteilhaft, wenn R₁, R₂ und/oder R₃ 16 bis 20 Kohlenstoffatome aufweisen und aus der Gruppe der einfach bis dreifach ungesättigten Carbonsäurereste gewählt werden.

Wenn R₁, R₂ und/oder R₃ Hydroxylgruppen tragen, ist der bevorzugte Alkenylcarboxylrest der Ricinolsäurerest.

Besonders vorteilhaft ist auch, Öle aus der Gruppe Sojaöl, Sonnenblumenöl, Weizenkeimöl und Ricinusöl zu wählen.

Überraschend hat sich gezeigt, daß gerade der Einsatz des kationischen Filmbildners Chitosan im Bereich zwischen 0,1 bis 5,0 % - vorzugsweise zwischen 0,5 bis 2,5 Gew.-% - in Kombination mit den Silikonemulgatoren Cetyldimethiconcopolyol sowie Laurylmethiconcopolyol in einer vorzugsweise aus mindestens 10 Gew.-%, bezogen auf die Gesamtzusammensetzung, aus Silikonölen bestehenden Lipidphase (Dimethicon unterschiedlicher Kettenlänge sowie Cyclomethicon, Anteil an Silikonöl > 50 % bezogen auf Gesamtgewicht der Fettphase) zu langzeitstabilen Wasser-in-Silikon Emulsionen mit sehr guten sensorischen Eigenschaften (seidiges, leichtes Hautgefühl in Kombination mit einem angenehm trockenen, pudrigen Rückstand auf der Haut) führt. Die geringe Klebrigkeit und das leichte, seidige Hautgefühl bleiben auch bei Verwendung höherer Einsatzkonzentrationen (> 10 %) an Moisturizern wie z.B. Glycerin sowie bei Zusatz weiterer unpolarer Lipide wie z.B. Mineralöl, Vaseline und Dicaprylyl Ether (anteilig bis max. 50 % bezogen auf Gesamtgewicht der Fettphase) erhalten.

Die wäßrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, oder auch der Typen ETD (Easy-to-disperse) 2001, 2020, 2050, jeweils einzeln oder in beliebigen Kombinationen untereinander.

Ein weiterer besonderer Vorzug der vorliegenden Erfindung ist es, daß sie gestattet, hohe Konzentrationen an Polyolen, insbesondere Glycerin einzusetzen.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Ψ-Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z. B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin E-Acetat), Vitamin A und Derivate (Vitamin A-palmitat) sowie Konyferylbenzoat des Benzoëharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Seien und dessen Derivate (z.B. Selenmethionin), Stilbene und deren Derivate (z.B. Stilbenoxid, Trans-Stilbenoxid) und die erfindungsgemäß geeigneten Derivate (Satze, Ester, Ether, Zucker, Nukleotide, Nukleoside, Peptide und Lipide) dieser genannten Wirkstoffe.

Besonders vorteilhaft im Sinne der vorliegenden Erfindung können öllösliche Antioxidantien eingesetzt werden.

Eine erstaunliche Eigenschaft der vorliegenden Erfindung ist, daß erfindungsgemäße Zubereitungen sehr gute Vehikel für kosmetische oder dermatologische Wirkstoffe in die Haut sind, wobei bevorzugte Wirkstoffe Antioxidantien sind, welche die Haut vor oxidativer Beanspruchung schützen können. Bevorzugte Antioxidantien sind dabei Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Es ist dem Fachmann natürlich bekannt, daß kosmetische Zubereitungen zumeist nicht ohne die üblichen Hilfs- und Zusatzstoffe denkbar sind. Die erfindungsgemäßen kosmetischen und dermatologischen Zubereitungen können dementsprechend ferner kosmetische Hilfsstoffe enthalten, wie sie üblicherweise in solchen Zubereitungen verwendet werden, beispielsweise Konsistenzgeber, Stabilisatoren, Füllstoffe, Konservierungsmittel, Parfüme, Substanzen zum Verhindern des Schäumens, Farbstoffe, Pigmente, die färbende Wirkung haben, Verdickungsmittel, oberflächenaktive Substanzen, Emulgatoren, weichmachende, anfeuchtende und/oder feuchthaltende Substanzen, entzündungshemmende Substanzen, zusätzliche Wirkstoffe wie Vitamine oder Proteine, Lichtschutzmittel, Insektenrepellentien, Bakterizide, Viruzide, Wasser, Salze, antimikrobiell, proteolytisch oder keratolytisch wirksame Substanzen, Medikamente oder andere übliche Bestandteile einer kosmetischen oder dermatologischen Formulierung wie Alkohole, Polyole, Polymere, Schaumstabilisatoren, organische Lösungsmittel oder auch Elektrolyte.

Letztere können beispielsweise gewählt werden aus der Gruppe der Salze mit folgenden Anionen: Chloride, ferner anorganische Oxo-Element-Anionen, von diesen insbesondere Sulfate, Carbonate, Phosphate, Borate und Aluminate. Auch auf organischen Anionen basierende Elektrolyte sind vorteilhaft, z.B. Lactate, Acetate, Benzoate, Propionate, Tartrate, Citrate, Aminosäuren, Ethylendiamintetraessigsäure und deren Salze und andere mehr. Als Kationen der Salze werden bevorzugt Ammonium,- Alkylammonium,- Alkalimetall-, Erdalkalimetall,- Magnesium-, Eisen- bzw. Zinkionen verwendet. Es bedarf an sich keiner Erwähnung, daß in Kosmetika nur physiologisch unbedenkliche Elektrolyte verwendet werden sollten. Besonders bevorzugt sind Kaliumchlorid, Kochsalz, Magnesiumsulfat, Zinksulfat und Mischungen daraus.

Mutatis mutandis gelten entsprechende Anforderungen an die Formulierung medizinischer Zubereitungen.

Die erfindungsgemäßen W/O-Emulsionen können als Grundlage für kosmetische oder dermatologische Formulierungen dienen. Diese können wie üblich zusammengesetzt sein und beispielsweise zur Behandlung und der Pflege der Haut und/oder der Haare, als Lippenpflegeprodukt, als Deoprodukt und als Schmink- bzw. Abschminkprodukt in der dekorativen Kosmetik oder als Lichtschutzpräparat dienen. Zur Anwendung werden die erfindungsgemäßen kosmetischen und dermatologischen Zubereitungen in der für Kosmetika oder Dermatika üblichen Weise auf die Haut und/oder die Haare in ausreichender Menge aufgebracht.

Entsprechend können kosmetische oder topische dermatologische Zusammensetzungen im Sinne der vorliegenden Erfindung, je nach ihrem Aufbau, beispielsweise verwendet werden als Hautschutzcrème, Reinigungsmilch, Sonnenschutzlotion, Nährcrème, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Die dünnflüssigen kosmetischen oder dermatologischen Mittel gemäß der Erfindung können beispielsweise als aus Aerosolbehältern, Quetschflaschen oder durch eine Pumpvorrichtung versprühbare Präparate vorliegen oder in Form einer mittels Roll-on-Vorrichtungen auftragbaren flüssigen Zusammensetzung, jedoch auch in Form einer aus normalen Flaschen und Behältern auftragbaren Emulsion.

Als Treibmittel für aus Aerosolbehältern versprühbare kosmetische oder dermatologische Zubereitungen im Sinne der vorliegenden Erfindung sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Günstig sind auch solche kosmetischen und dermatologischen Zubereitungen, die in der Form eines Sonnenschutzmittels vorliegen. Vorzugsweise enthalten diese neben den erfindungsgemäßen Wirkstoffkombinationen zusätzlich mindestens eine UV-A-Filtersubstanz und/oder mindestens eine UV-B-Filtersubstanz und/oder mindestens ein anorganisches Pigment.

Es ist aber auch vorteilhaft im Sinne der vorliegenden Erfindungen, solche kosmetischen und dermatologischen Zubereitungen zu erstellen, deren hauptsächlicher Zweck nicht der Schutz vor Sonnenlicht ist, die aber dennoch einen Gehalt an UV-Schutzsubstanzen enthalten. So werden z.B. in Tagescrèmes gewöhnlich UV-A- bzw. UV-B-Filtersubstanzen eingearbeitet.

Auch stellen UV-Schutzsubstanzen, ebenso wie Antioxidantien und, gewünschtenfalls, Konservierungsstoffe, einen wirksamen Schutz der Zubereitungen selbst gegen Verderb dar.

Vorteilhaft können erfindungsgemäße Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Enthalten die erfindungsgemäßen Emulsionen UVB-Filtersubstanzen, können diese öllöslich oder wasserlöslich sein. Erfindungsgemäß vorteilhafte öllösliche UVB-Filter sind z.B.:
- 3-Benzylidencampher-Derivate, vorzugsweise 3-(4-Methylbenzyliden)campher, 3-Benzylidencampher;
- 4-Aminobenzoësäure-Derivate, vorzugsweise 4-(Dimethy(amino)-benzoësäure-(2-ethylhexyl)ester, 4-(Dimethylamino)benzoësäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester,
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester,
- Derivate des 1,3,5-Triazins, vorzugsweise 2,4,6-Trianilino-(p-carbo-2'-ethyl-1'-hexyloxy)-1,3,5-triazin.

Die Liste der genannten UVB-Filter, die in Kombination mit den erfindungsgemäßen Wirkstoffkombinationen verwendet werden können, soll selbstverständlich nicht limitierend sein.

Es kann auch von Vorteil sein, erfindungsgemäße Lipodispersionen mit UVA-Filtern zu formulieren, die bisher üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion.

Erfindungsgemäße kosmetische und dermatologische Zubereitungen können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen, bei denen die Oxide die aktiven Agentien sind. Besonders bevorzugt handelt es sich um Pigmente auf der Basis von Titandioxid.

Als weitere Bestandteile können verwendet werden:
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Erfindungsgemäße Zubereitungen können auch Wirkstoffe (eine oder mehrere Verbindungen) enthalten, welche gewählt werden aus der Gruppe: Acetylsalicylsäure, Atropin, Azulen, Hydrocortison und dessen Derivaten, z.B. Hydrocortison-17-valerat, Vitamine, z.B. Ascorbinsäure und deren Derivate, Vitamine der B- und D-Reihe, sehr günstig das Vitamin B₁, das Vitamin B₁₂ das Vitamin D₁, aber auch Bisabolol, ungesättigte Fettsäuren, namentlich die essentiellen Fettsäuren (oft auch Vitamin F genannt), insbesondere die γ-Linolensäure, Ölsäure, Eicosapentaënsäure, Docosahexaënsäure und deren Derivate, Chloramphenicol, Coffein, Prostaglandine, Thymol, Campher, Extrakte oder andere Produkte pflanzlicher und tierischer Herkunft, z.B. Nachtkerzenöl, Borretschöl oder Johannisbeerkernöl, Fischöle, Lebertran aber auch Ceramide und ceramidähnliche Verbindungen und so weiter. Vorteilhaft ist es auch, die Wirkstoffe aus der Gruppe der rückfettenden Substanzen zu wählen, beispielsweise Purcellinöl, Eucerit® und Neocerit®.

Die Menge solcher Wirkstoffe (eine oder mehrere Verbindungen) in den Zubereitungen gemäß der Erfindung beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

### Beispiel 1 (W/O-Creme):

| | Gew.-% |
|---|---|
| Cetyldimethiconcopolyol¹ | 1,5 |
| Caprylsäure/Caprinsäuretriglyceride | 4.0 |
| Dicaprylylether | 3,0 |
| Octyldodecanol | 3.0 |
| Cyclomethicon | 10,0 |
| Glycerin | 7,0 |
| Natriumchlorid | 0,7 |
| Chitosan (Chitopure 026-2)² | 0,25 |
| Milchsäure (90%ig) | 0,2 |
| Parfüm, Konservierungsmittel, Farbstoffe, Antioxidantien etc. | q.s. |
| Wasser | ad. 100,0 |

| | |
|---|---|
| ¹ ABIL EM 90 (Th. Goldschmidt) | |
| ² Fish Contract | |

### Beispiel 2 (W/O-Creme):

| | Gew.-% |
|---|---|
| Laurylmethiconcopolyol³ | 2,0 |
| Octyldodecanol | 1,0 |
| C₁₂₋₁₅ Alkylbenzoate | 1,0 |
| Squalan | 1,0 |
| Paraffinum liquidum | 6,0 |
| Cyclomethicon | 12,0 |
| Dimethicon | 3,5 |
| Glycerin | 5,0 |
| Natriumchlorid | 0,7 |
| Chitosan (Hydagen HMFP)⁴ | 0,75 |
| Milchsäure (90%ig) | 0,6 |
| Parfüm, Konservierungsmittel, Farbstoffe, Antioxidantien etc. | q.s. |
| Wasser | ad. 100,0 |

| | |
|---|---|
| ³ DC Q2-5200 Formulation Aid (Dow Corning) | |
| ⁴ Henkel | |

### Beispiel 3 (W/O-Creme):

| | Gew.-% |
|---|---|
| Cetyldimethiconcopolyol | 2,0 |
| Squalan | 2,0 |
| Paraffinum liquidum | 3,0 |
| Stearylheptanoat | 1,0 |
| Hydriertes Polysiobuten | 3,0 |
| Cyclomethicon | 12,0 |
| Dimethicon | 2,0 |
| Glycerin | 3,0 |
| Natriumchlorid | 0,7 |
| Chitosan (TM37)⁵ | 1,25 |
| Milchsäure (90%ig) | 1,0 |
| Parfüm, Konservierungsmittel, Farbstoffe, Antioxidantien etc. | q.s. |
| Wasser | ad. 100,0 |

| | |
|---|---|
| ⁵ Primex | |

### Beispiel 4 (W/O-Lotion):

| | Gew.-% |
|---|---|
| Laurylmethiconcopolyol | 2,0 |
| Paraffinum liquidum | 15,0 |
| Cyclomethicon | 12,0 |
| Dimethicon | 3,0 |
| Tocopherolacetat | 0,5 |
| Glycerin | 3,0 |
| Panthenol | 0,3 |
| 1,3 Butylenglycol | 1,0 |
| Serin | 0,3 |
| Biotin | 0,1 |
| Distärkephosphat | 1,0 |
| Natriumchlorid | 0,7 |
| Chitosan (SC 142)⁶ | 0,3 |
| Chitosan (SC 242)⁷ | 0,6 |
| Chitosan (SC 342)⁸ | 0,3 |
| Milchsäure (90%ig) | 1,0 |
| Parfüm, Konservierungsmittel, Farbstoffe, Antioxidantien etc. | q.s. |
| Wasser | ad. 100,0 |

| | |
|---|---|
| ⁶ Natural Biopolymer | |
| ⁷ Natural Bioploymer | |
| ⁸ Natural Biopolymer | |

### Beispiel 5 (W/O-Creme):

| | Gew.-% |
|---|---|
| Cetyldimethiconcopolyol | 2,0 |
| Isohexadecan | 4,0 |
| Paraffinum liquidum | 7,0 |
| Cyclomethicon | 11,5 |
| Dimethicon | 0,5 |
| Butylmethoxydibenzoylmethan | 1,0 |
| Octyl Methoxycinnamate | 2,0 |
| Methylbenzylidencampher | 1,5 |
| Octyltriazon | 0,5 |
| Titandioxid | 1,0 |
| Zinkoxid | 1,0 |
| Glycerin | 1,0 |
| Natriumchlorid | 0,7 |
| Chitosan (Dermosoft OSAN 40)⁹ | 0,8 |
| Milchsäure (90%ig) | 0,7 |
| Parfüm, Konservierungsmittel, Farbstoffe, Antioxidantien etc. | q.s. |
| Wasser | ad. 100,0 |

| | |
|---|---|
| ⁹ Straetmann | |

### Beispiel 6 (W/O-Creme):

| | Gew.-% |
|---|---|
| Cetyldimethiconcopolyol | 2,5 |
| Caprylsäure/Caprinsäuretriglyceride | 4.0 |
| Dicaprylylether | 6,5 |
| Octyldodecanol | 5.5 |
| Cyclomethicon | 7,5 |
| Glycerin | 30,0 |
| Propyleneglycol | 5,0 |
| Natriumchlorid | 0,7 |
| Chitosan (SC 242)¹⁰ | 1,0 |
| Milchsäure (90%ig) | 0,9 |
| Parfüm, Konservierungsmittel, Farbstoffe, Antioxidantien etc. | q.s. |
| Wasser | ad. 100,0 |

| | |
|---|---|
| ¹⁰ Natural Biopolymer | |

### Beispiel 7 (W/O-Creme):

| | Gew.-% |
|---|---|
| Laurylmethiconcopolyol | 1,0 |
| Cetyldimethiconcopolyol | 1,0 |
| Sonnenblumenöl | 4,0 |
| Dicaprylylether | 5,0 |
| Paraffinum liquidum | 6,0 |
| Cyclomethicon | 8,5 |
| Dimethicon | 0,5 |
| Dimethiconol | 1.0 |
| Phenyltrimethicon | 2,0 |
| Glycerin | 3,0 |
| Natriumchlorid | 0,7 |
| Chitosan (TM37)¹¹ | 1,5 |
| Milchsäure (90%ig) | 1,2 |
| Parfüm, Konservierungsmittel, Farbstoffe, Antioxidantien etc. | q.s. |
| Wasser | ad. 100,0 |

| | |
|---|---|
| ¹¹ Primex | |

### Beispiel 8 (W/S-Creme):

| | Gew.-% |
|---|---|
| Laurylmethiconcopolyol | 2,5 |
| Cyclomethicon | 23,0 |
| Dimethicon | 2,0 |
| Sorbitol | 15,0 |
| Natriumchlorid | 0,7 |
| Chitosan (Dermosoft OSAN 40)¹² | 1,3 |
| Milchsäure (90%ig) | 1,0 |
| Parfüm, Konservierungsmittel, Farbstoffe, Antioxidantien etc. | q.s. |
| Wasser | ad. 100,0 |

| | |
|---|---|
| ¹² Straetmann | |

### Beispiel 9 (W/S-Lotion):

| | Gew.-% |
|---|---|
| Cetyldimethiconcopolyol | 2,0 |
| Cyclomethicon | 25,0 |
| Dimethicon | 2,5 |
| Dimethiconol | 0,5 |
| Dimethiconcopolyol | 0,3 |
| Glycerin | 12,5 |
| Natriumchlorid | 0,7 |
| Chitosan (Chitopure 026-2)¹³ | 0,6 |
| Milchsäure (90%ig) | 0,5 |
| Parfüm, Konservierungsmittel, Farbstoffe, Antioxidantien etc. | q.s. |
| Wasser | ad. 100,0 |

| | |
|---|---|
| ¹³ Fish Contract | |

### Beispiel 10 (Emulsions-Make-up):

| | Gew.-% |
|---|---|
| Cetyldimethiconcopolyol | 1,5 |
| Octyldodecanol | 2,0 |
| C₁₂₋₁₅ Alkylbenzoate | 2,0 |
| Squalan | 1,0 |
| Paraffinum liquidum | 1,0 |
| Distärkephosphat | 0,5 |
| Cyclomethicon | 8.0 |
| Dimethicon | 0,5 |
| Glycerin | 1,5 |
| Chitosan (SC 142)¹⁴ | 0,2 |
| Chitosan (SC 242)¹⁵ | 0,2 |
| Chitosan (SC 342)¹⁶ | 0,4 |
| Milchsäure (90%ig) | 0,5 |
| Magnesiumsilikat | 1,0 |
| Glimmer | 0,5 |
| Eisenoxide | 0,5 |
| Titandioxid | 1,0 |
| Talkum | 1,0 |
| Tapiocastärke | 0,25 |
| Parfüm, Konservierungsmittel, NaOH, Farbstoffe, Antioxidantien etc. | q.s. |
| Wasser | ad 100,0 |

| | |
|---|---|
| ¹⁴ Natural Biopolymer | |
| ¹⁵ Natural Biopolymer | |
| ¹⁶ Natural Biopolymer | |

### Allgemeine Anmerkungen zu den Beispielrezepturen:

1) pH-Wert der Wasserphase der Emulsion durch Zugabe von Säuren, die mit Chitosan in Wasser lösliche Salze bilden (wie z.B. Milchsäure, Glykolsäure, Essigsäure, Salicylsäure, Asparaginsäure) auf pH_{Wasserphase} < 6,0 (vorzugsweise 3,5 bis 5,5) einstellen.
2) Chitosan: mittleres Molekulargewicht von 50.000 bis 2.000.000 g/mol [bestimmt mittels Gelpermeationschromatographie] und einem Deacylierungsgrad von 10 bis 99 % [bestimmt mittels ¹H-NMR].

## Patentansprüche

1. Wasser-in-Öl-Emulsionen
(a) eines Gehaltes an Wasser und gegebenfalls wasserlöslichen Substanzen
(b) und eines Gehaltes an Lipiden und gegebenenfalls lipophilen Bestandteilen von insgesamt mindestens 20 Gew.-%, jeweils bezogen auf das Gesamtgewicht der Zubereitungen,
enthaltend:
(c) wenigstens ein Siliconöl in einem Gehalt von mindestens 7,5 Gew.-%, bevorzugt mindestens 10 Gew.-%, bezogen auf das Gesamtgewicht der Zubereitungen
(d) mindestens eine grenzflächenaktive Substanz, gewählt aus der Gruppe der Alkylmethiconcopolyole und/oder Alkyl-Dimethiconcopolyole,
(e) ein oder mehrere kationische Polymere.

2. Emulsionen nach Anspruch 1, dadurch gekennzeichnet, daß als grenzflächenaktive Substanz Cetyldimethiconcopolyol gewählt wird.

3. Emulsionen nach Anspruch 1, dadurch gekennzeichnet, daß als grenzflächenaktive Substanz das Laurylmethiconcopolyol gewählt wird.

4. Emulsionen nach Anspruch 1, dadurch gekennzeichnet, daß die Gesamtmenge an den Alkylmethiconcopolyolen und/oder Alkyl-Dimethiconcopolyolen aus dem Bereich von 0,075 - 7,5 Gew.-%, bevorzugt 0,1- 5,0 Gew.-%, insbesondere 1,0 - 3,0 Gew.-% gewählt, bezogen auf das Gesamtgewicht der Zubereitungen.

5. Emulsionen nach Anspruch 1, dadurch gekennzeichnet, daß sie 0,01 bis 10%, bevorzugt 0,25 - 1,25 % an kationischen Polymeren enthalten.

6. Emulsionen nach Anspruch 1, dadurch gekennzeichnet, daß das oder die kationischen Polymere gewählt werden aus der Gruppe der kationische Cellulosederivate, kationische Stärke, Copolymere von Diallylammoniumsalzen und Acrylamiden, quaternisierte Vinylpyrrolidon/Vinyl-imadazol-Polymere, Kondensationsprodukte von Polyglycolen und Aminen, quaternisierte Kollagenpolypeptide, quaternisierte Weizenpolypeptide, Polyethylenimin, kationische Silikonpolymere, Copolymere der Adipinsäure mit Dimethylaminohydroxypropyldietylentriamin, Copolymere der Acrylsäure mit Dimethyldiallylamoniumchlorid, Polyaminopolyamide, kationische Chitinderivate, kationischer Guar-Gum, quaternisierte Ammoniumsalz-Polymere sowie kationische Biopolymere wie z.B. Chitosan (mittleres Molekulargewicht von 50.000 bis 2.000.000 g/mol [bestimmt mittels Gelpermeationschromatographie] und einem Deacylierungsgrad von 10 bis 99% [bestimmt mittels ¹H-NMR]).
